# EUROPEAN PATENT SPECIFICATION

(11) **EP 4 248 845 B1**
(45) Date of publication and mention of the grant of the patent: **14.01.2026**
(21) Application number: 23163404.9
(22) Date of filing: 22.03.2023
(51) Int. Cl.: A61B 5/00, A61B 5/024, A61B 5/08, A61B 5/11

(54) **PATIENT MONITORING SYSTEM**
PATIENTENÜBERWACHUNGSSYSTEM
SYSTÈME DE SURVEILLANCE DE PATIENT

(30) Priority: 24.03.2022 NL 2031404
(43) Date of publication of application: 27.09.2023
(73) Proprietor: Deron B.V., 6691 EE Gendt (NL)
(72) Inventor: GRIMBERG, Wilfried, 6691 MC Gendt (NL)
(74) Representative: Brantsandpatents bv

(56) References cited:
- EP-A1- 2 705 790
- CN-A- 108 095 418
- CN-A- 111 513 722
- CN-A- 113 041 059
- JP-A- 2020 089 668
- US-A1- 2016 136 385

## Description

### FIELD OF THE INVENTION

The present invention relates generally to a field of patient monitoring systems, and more particularly for detecting the position of the patient on a mattress to prevent development of decubitus ulcers and/or to prevent a patient from falling out or exiting a bed on which the mattress resides.

### BACKGROUND

In clinical settings, such as hospitals, nursing homes, geriatric care homes, and the like, patients or old people remain confined to the bed for long durations of time. Such people may either be affected with mobility problems or other disability or may be unconscious for long durations of time, therefore, special care needs to be provided else they may develop other issues such as body sores, etc. There are remote monitoring devices, which assist the hospital staff in monitoring such patients and recommending timely intervention.

One such a device/composition is also known from US7656299. US '299 describes a medical monitoring system for providing an indication of whether a patient in a hospital, nursing home, assisted living facility or other healthcare facility, is in bed, out of bed, or is actively attempting to exit the bed based on the position of the patient from the edge of the bed. A specific lay-out and combination of sensors is provided in order to achieve this. However, as the sensors are not incorporated in a mattress, but are affixed to the inside of the top surface of a mattress coverlet, the position of the sensors can easily shift, making the measurements less accurate. In addition, no sensors are present lining the lateral edges of the mattress/bed.

US8672842B2 describes a smart mattress for real-time medical monitoring of individuals such as hospital patients, bed-ridden patients, and infants susceptible to Sudden Infant Death Syndrome (SIDS). The mattress is capable of measuring and reporting (locally or remotely) a patient's vitals and other information to a server and/or caregiver during emergency and non-emergency situations. The mattress comprises a sensor pad which is affixed on a top surface of the mattress and comprises a matrix array of plural pressure sensors. As such, and similar to US '299 described above, the sensors are not incorporated in the mattress. US '842 further describes a piezo-electric sensor, however, this sensor is also not incorporated in the mattress, but is mounted on the patient.

US20200390403A1 describes a system and method for monitoring patients and predicting abnormal physiological conditions. US '403 describes a motion sensor configured to sense motion of a subject on a bed without contacting or viewing the subject or clothes the subject is wearing. Further, US '403 discloses a weight sensor, configured to measure a weight of the subject on a portion of the bed and generate a weight signal which can be analyzed to identify an aspect of the motion signal indicative of a possible need to generate an alert. Such analysis verifies compliance of the pressure ulcer prevention protocol by utilizing the differentiation between body motion that involves a posture change and body motion that does not involve a posture change. However, said weight sensor is embedded in the bed and is not incorporated in the mattress itself, limiting the accuracy of the measurements.

CN211381368 describes an intelligent monitoring system based on a ballistocardiogram signal device to detect cardiac shock signals from a piezoelectric sensing mattress. The piezoelectric sensing mattress includes pressure sensors and BCG sensors to collect the motion information data of the subject, and to collect the heart shock signal data of subjects to determine heart rate, breathing, and body movement signals. CN '368 further describes that the pressure sensor is fixed on the piezoelectric sensing mattress and that the subject's shoulders, chest, abdomen, buttocks and other positions are in contact with the pressure sensor. The subject's spine is in contact with the BCG sensor and the sensor can be attached to the clothes normally worn by the subject. However, no details regarding the specific position and nature of the sensors is provided by CN '368. JP2020089668 describes a bioinformation detecting device of a bed mattress.

The present invention aims to provide a patient monitoring system and a mattress for patient monitoring, which allows to accurately determine posture changes of apatient and thereby mitigating the risk of development of bed sores owing to being in the same position for long durations of time. Further, the invention allows to detect and signal when a patient is exiting the bed on which the mattress resides or when a patient is at risk of falling out of said bed. The present invention further aims to provide an optimal transmission of the sensor data for further processing.

### SUMMARY OF THE INVENTION

The present invention and embodiments thereof serve to provide a solution to one or more of above-mentioned disadvantages. To this end, the present invention relates to a mattress according to claim 1. More specifically, the present invention relates to a patient monitoring system, the system comprising:
a mattress **1,** the mattress **1** having a first edge **6** and a second edge **15,** wherein the first edge **6** and the second edge **15** are spaced apart from each other along a longitudinal axis **10** of the mattress **1,** the mattress **1** comprising a sensor assembly, said sensor assembly comprising:
   - a plurality of sensors disposed in the mattress, wherein the plurality of sensors transmit signals and comprise:
      one or more piezoelectric sensor strips **3;**
      a sensor grid **4** comprising a plurality of discrete pressure sensors **16** arranged in one or more rows **11,12,13,** each of the one or more rows **11,12,13** being coupled with each other through a conducting element, wherein the one or more piezoelectric sensor strips **3** and the sensor grid **4** are disposed in proximity to the first edge **6** of the mattress **1;**
      one or more linearly extending pressure sensors **5a,5b** extending along at least a part length of or a full length of the mattress **1,** wherein the one or more linearly extending pressure sensors **5a,5b** are placed in parallel with and in proximity of a third edge **7** and/or a fourth edge **8** of the mattress **1,** wherein the third edge **7** and the fourth edge **8** of the mattress **1** are spaced apart from each other along a lateral axis **9** of the mattress **1,** said linearly extending pressure sensors **5a,5b** covering at least 50% of the length of the lower half of the mattress **1,** measured according to the longitudinal axis **10** of the mattress **1;**
   - a sensor module **2** for receiving said signals from said sensors **3,4,5,** wherein the sensor module **2** is mounted within the mattress **1,**
a control unit electrically coupled to the sensor module **2** for processing said signals, and
at least one monitoring station communicatively coupled to the control unit.

Preferred embodiments of the mattress are shown in any of the claims 2 to 6.

Claim 2 describes mattress according to claim 1, wherein the sensor grid **4** comprises at least 12 discrete pressure sensors **16** arranged in one or more rows, preferably 16 discrete pressure sensors **16,** arranged in a first **11,** second **12** and third row **13,** said rows **11,12,13** extending laterally across a part of or across a total width of the mattress **1,** the first row **11** being closest to the first edge **6** of the mattress **1,** the second row **12** being placed in between the first **11** and third row **13** and the third row **13** being closest to the second edge **15** of the mattress, the three rows **11,12,13** comprising respectively 7, 6 and 3 discrete pressure sensors **16** and wherein the pressure sensors **16** of the first row **11** are arranged in such a manner that the pressure sensor **16** closest to the third edge **7** and the pressure sensor closest to the fourth edge **8** are spaced a minimal distance apart, said minimal distance covering at least 60% of the width of the mattress **1** measured according to the lateral axis **9** of the mattress **1.**

Claim 3 describes the mattress according to claim 2, the mattress **1** comprising one piezoelectric sensor strip **3** extending laterally across a part of or across a total width of the mattress **1,** said piezoelectric sensor strip **3** being located between the second **12** and third row **13** of the sensor grid **4** and covering at least 65% of the width of the mattress **1** measured according to the lateral axis **9** of the mattress **1.**

Claim 4 describes the mattress according to one of the previous claims, wherein the mattress **1** comprises a first layer and one or more additional layers, the first layer being the top layer of the mattress being closest to the patient when the latter is present, wherein the plurality of sensors **3,4,5** are disposed between said layers of the mattress.

Claim 5 describes the mattress of claim 4, wherein the thickness of the first layer and the joint thickness of the one or more additional layers relate to each other with a ratio comprising between 45% and 80%.

Claim 6 describes above-mentioned mattress, wherein the plurality of sensors **3,4,5** further comprises one or more temperature sensors.

Claim 8 describes the above-mentioned patient monitoring system, wherein the control unit is configured to generate an alarm signal based on data received from the plurality of sensors.

Claim 9 describes the above-mentioned patient monitoring system, wherein the control unit is communicatively coupled to a remote monitoring station by means of a low-power wide-area-network, such as an LTE-M module.

In an other aspect the disclosure relates to a method.

More specifically, the disclosure relates to a method for monitoring a patient using the patient monitoring system or the mattress, the method comprises:
a. receiving piezoelectric sensor data from one or more piezoelectric sensor strips **3** indicating body motion of the patient;
b. receiving force sensor data from the sensor grid **4** indicating a position of the patient on the mattress;
c. differentiation between body motion that involves a posture change of the patient and body motion that does not involve a posture change of the patient based on the piezoelectric sensor data and the force sensor data; and
d. generating an alarm signal when no posture change has occurred during a time greater than a certain threshold.

In an aspect, the above-mentioned method further comprising determining physiological parameters of the patient based on the piezoelectric sensor data from one or more of the piezoelectric sensor strips **3.**

In an aspect, the above-mentioned method, wherein said physiological parameters are used to verify the position of a patient.

In an aspect, the above-mentioned method, wherein said physiological parameters are used to diagnose breathing disorders, such as obstructive sleep apnea (OSA) syndrome.

### DESCRIPTION OF FIGURES

The following description of the figures of specific embodiments of the invention is merely exemplary in nature and is not intended to limit the present teachings, their application or uses. Throughout the drawings, corresponding reference numerals indicate like or corresponding parts and features.

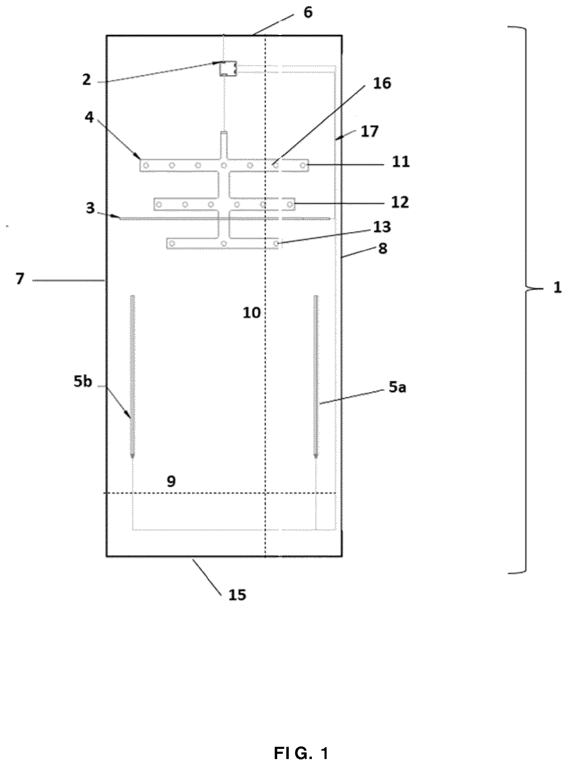

**Figure 1** shows a schematic layout of the position of the various sensors with respect to the mattress of the patient monitoring system according to an embodiment of the invention.

### DETAILED DESCRIPTION OF THE INVENTION

The present invention concerns a mattress for patient monitoring and a patient monitoring system. In addition, a method for patient monitoring using the above-mentioned patient monitoring system and/or mattress is disclosed.

The present invention allows to accurately determine the position of the patient. Further, the invention aims to provide an optimal transmission of the sensor data for further processing, thereby mitigating the risk of development of bed sores owing to being in the same position for long durations of time.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g.,* any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In a first aspect, the invention relates to a patient monitoring system (PMS), the system comprising:
- a mattress, the mattress having a first edge and a second edge, wherein the first edge and the second edge are spaced apart from each other along a longitudinal axis of the mattress, the mattress comprising a sensor assembly, said sensor assembly comprising:
   - a plurality of sensors disposed in the mattress, wherein the plurality of sensors transmit signals and comprise:
      one or more piezoelectric sensor strips;
      a sensor grid comprising a plurality of discrete pressure sensors arranged in one or more rows, each of the one or more rows being coupled with each other through a conducting element, wherein the one or more piezoelectric sensor strips and the sensor grid are disposed in proximity to the first edge of the mattress;
      one or more linearly extending pressure sensors extending along at least a part length of or a full length of the mattress, wherein the one or more linearly extending pressure sensors are placed in parallel with and in proximity of a third edge and/or a fourth edge of the mattress, wherein the third edge and the fourth edge of the mattress are spaced apart from each other along a lateral axis of the mattress, said linearly extending pressure sensors covering at least 50% of the length of the lower half of the mattress measured according to the longitudinal axis of the mattress;
   - a sensor module for receiving said signals from said sensors, wherein the sensor module is mounted within the mattress,
- a control unit electrically coupled to the sensor module for processing said signals, and
- at least one monitoring station communicatively coupled to the control unit.

Said first edge is located in proximity to a headboard of a bed and said second edge is located in proximity towards a footboard of a bed. Further, the longitudinal axis extends along the length of the mattress. The lateral axis extends along the breadth of the mattress.

By providing a PMS according to the invention, having above-mentioned configuration and location of the plurality of sensors, more specifically, having one or more piezoelectric sensor strips; a sensor grid comprising a plurality of discrete pressure sensors and a plurality of linearly extending pressure sensors in their respective locations, allows for an accurate determination of patient movement and of the position of a patient residing on the mattress of the PMS, making it possible to detect changes in the posture of a patient residing on the mattress of the PMS.

In an embodiment, reliable in-bed detection is provided by the plurality of sensors. By using multiple sensors at discrete locations along the length and width of the bed, and redundant detection of sensor signals to confirm an in-bed condition, the patient is able to move around normally in bed and assume a variety of different positions in the bed without activating an alarm, and there is a minimum risk of a false in-bed determination.

In a preferred embodiment, said piezoelectric sensor strips allow to detect body movement of the patient residing on the mattress.

In an embodiment, said piezoelectric sensor strips allow to detect other signals, such as heartbeat-induced repeated motions of the human body (measured by a technique called ballistocardiography) and changes in load or center of gravity due to breathing of the patient. In an embodiment, said signals when assessed over an extended time period may be indicative of the position of a patient, whereas a high breathing amplitude and heart rate of a patient are indicative for abdominal position of the patient, a median breathing amplitude and high heart rate are indicative for left side position of the patient and a median breathing amplitude and low heart rate are indicative for a rightside position of the patient. In an embodiment, said piezoelectric sensor data may further be used to monitor sleeping pattern of the patient that further includes duration, a phase, or a quality of sleep of the patient. In an embodiment, said signals from the one or more piezoelectric sensor strips are used to detect breathing disorders, such as sleep-disordered breathing (SDB). SDB, better known as the obstructive sleep apnea (OSA) syndrome, and associated cardiovascular complications are among the most common clinical disorders. The ballistocardiogram (BCG) signal measured by the one or more piezoelectric sensor strips captures the ballistic forces of the heart caused by the sudden ejection of blood into the great vessels with each heartbeat, breathing, and body movement. In an embodiment, cardiac function and performance is measured by the one or more piezoelectric strips.

In an embodiment, the sensor grid includes a plurality of discrete pressure sensors which are arranged in one or more rows. In a preferred embodiment, all the discrete pressure sensors converge on a single connector, extending the product life of the sensor grid (and consequently the product life of the mattress and the PMS) and the time needed to assemble the mattress (and PMS). In an embodiment, the sensor grid is disposed in proximity to the third edge of the mattress, and extends laterally across a part of or across the total width of the mattress towards the fourth edge along the lateral axis. In an embodiment, the sensor grid is an array of force sensing resistors which are spread in such a manner that a torso of the patient lying on the mattress overlaps one or more of the plurality of discrete pressure sensors.

In some examples, the sensor grid includes one or more strips that are positioned in a reverse pyramidal structure such that length of each strip is different. Further, the length of each of the one or more strips reduces as is observed from the first edge to the second edge. In some examples, the varying length of the one or more strips enables capturing of the anatomical shape of the torso of the patient. Further, each of the one or more strips are electrically coupled with each other through a conductive element that extends along the longitudinal axis of the mattress.

In an embodiment, said sensor grid comprising a plurality of discrete pressure sensors allows to collect information about pressure points in the torso region after which an algorithm gives a position classification. In an embodiment, said classification will be directly linked to a posture (the signals from the sensor grid when analyzed can for instance easily distinguish whether the patient is in a supine, prone or foetal posture). In an alternative embodiment, this classification will not be directly linked to a posture but is purely for determining whether there is a new posture measured after the piezo sensor indicates that a movement has taken place.

In an embodiment, the linearly extending pressure sensors extend along at least a part length of or full length of the mattress, and are positioned proximal to the lateral edges and of the mattress. The linearly extending pressure sensors (are capable of detecting the position of a patient in proximity to the lateral edges of the mattress. Said linearly extending pressure sensors allow to collect spatially relevant information about pressure points covering at least a part of the lateral edges of the lower halve of the bed, indicating whether a patient might be exiting the bed.

In an embodiment, the linearly extending pressure sensors may be force sensing resistors (FSR).

As such, the PMS also provides for reliable bed exit detection to determine if a patient is out of bed or has assumed a position or sequence of positions near the foot or sides of the bed, indicating a likelihood that the patient is in the process of exiting the bed. In at least one aspect of the invention, the separate bed exit detection is provided by said one or more linearly extending pressure sensors placed in parallel with and in proximity of a third edge and/or a fourth edge of the mattress and covering at least 50% of the length of the lower half of the mattress. By comprising linearly extending pressure sensors covering at least 50% of the length of the lower half of the mattress, the PMS is able to provide reliable bed exit detection. The system alarms promptly (within a few seconds) when a patient is completely out of bed, and in many cases, will alarm before the patient fully exits the bed. The bed exit alarm is user selectable (on/off), so that it can be turned off by the caregiver for patients who are not on bed restrictions.

Furthermore, the system allows the clinician to select the desired sensitivity level for each patient, allowing some patients more freedom of movement than others, before an alarm indication is provided. In some cases, the system can be set to alarm to prevent the patient from falling out of the bed (the patient is still in the bed, but too close to the edge of the mattress). In other cases, the system can be set to alarm to prevent the patient from wandering when the patient has fully exited the bed.

In some examples, the scope of the disclosure is not limited to the pressure sensors being FSR. In an exemplary embodiment, the pressure sensors may be implemented using any other known technologies.

Furthermore, by disposing the plurality of sensors in the mattress (and not affixed to the inside of the top surface of a mattress coverlet or affixed to the top surface of the mattress, or mounted on the patient, or affixed to the bed as disclosed in the cited prior art), the sensors do not do not provide any discomfort to the patient and are able to more accurately measure data and determine the position of the patient residing on the mattress of the PMS. Such accurate determination of position is important to detect the development of decubitus ulcers of a patient residing on the mattress of the PMS, to detect when a patient is at risk of falling out of the bed or to detect when a patient has exited the bed on which the mattress of the PMS resides. Furthermore, incorporation of the plurality of the sensors in the mattress allows for more easy handling of the sensors, minimizing the risk to damage or lose one or more of the provided sensors, extending the product life of the mattress (and the PMS).

The PMS is useable with different kinds and sizes of beds and with a wide range of patient populations. It can be used with small patients (e.g., approximately 100 pounds) or larger patients.

In an embodiment, said linearly extending pressure sensors cover at least 50%, more preferably at least 51%, more preferably at least 52%, more preferably at least 53%, more preferably at least 54%, more preferably at least 55%, more preferably at least 56%, more preferably at least 57%, more preferably at least 58%, more preferably at least 59%, more preferably at least 60%, such as 61% of the length of the lower half of the mattress measured according to the longitudinal axis of the mattress. By providing said linearly extending pressure sensors covering above-mentioned length of the lower half of the mattress, the PMS is able to collect spatially relevant information about pressure points covering the lateral edges of the lower halve of the bed, indicating whether a patient might be exiting the bed.

In a preferred embodiment of the PMS, the sensor grid comprises at least 12 discrete pressure sensors arranged in one or more rows, preferably 16 discrete pressure sensors, arranged in a first, second and third row, said rows extending laterally across a part of or across a total width of the mattress, the first row being closest to the first edge of the mattress, the second row being placed in between the first and third row and the third row being closest to the second edge of the mattress, the three rows comprising respectively 7, 6 and 3 discrete pressure sensors and the pressure sensors of the first row are arranged in such a manner that the pressure sensor closest to the third edge and the pressure sensor closest to the fourth edge are spaced a minimal distance apart, said minimal distance covering at least 60%, preferably at least 61%, preferably at least 62%, preferably at least 63%, preferably at least 64%, preferably at least 65%, such as 66%, of the width of the mattress measured according to the lateral axis of the mattress. In a preferred embodiment, the sensor grid may be an array of 16 force sensing resistors.

In a further embodiment, the pressure sensors of the second row are arranged in such a manner that the pressure sensor closest to the third edge and the pressure sensor closest to the fourth edge are spaced a minimal distance apart, said minimal distance covering at least 45%, preferably at least 46%, preferably at least 47%, preferably at least 48%, preferably at least 49%, preferably at least 50%, preferably at least 50%, preferably at least 52%, preferably at least 53%, preferably at least 54%, such as 55% of the width of the mattress measured according to the lateral axis of the mattress.

In a further embodiment, the pressure sensors of the third row are arranged in such a manner that the pressure sensor closest to the third edge and the pressure sensor closest to the fourth edge are spaced a minimal distance apart, said minimal distance covering at least 35%, preferably at least 36%, preferably at least 37%, preferably at least 38%, preferably at least 39%, preferably at least 40%, preferably at least 41%, preferably at least 42%, preferably at least 43%, such as 44% of the width of the mattress measured according to the lateral axis of the mattress.

By providing said specific configuration of pressure sensors covering above-mentioned width of the mattress, the PMS is able to collect spatially relevant information about pressure points covering the torso region, which allows for an accurate position classification of the patient residing on the mattress.

In a further embodiment, the mattress of the PMS comprises a single piezoelectric sensor strip extending laterally across a part of or across a total width of the mattress, said piezoelectric sensor strip being located between the second and third row of the sensor grid and covering at least 65%, preferably at least 66%, preferably at least 67%, preferably at least 68%, preferably at least 69%, preferably at least 70%, preferably at least 71%, preferably at least 72%, preferably at least 73%, preferably at least 74%, preferably at least 75%, preferably at least 76%, such as 77% of the width of the mattress measured according to the lateral axis of the mattress. By providing said piezoelectric sensor strip covering above-mentioned width of the mattress, the PMS is able to collect spatially relevant information about pressure points covering the torso region, which allows for an accurate position classification (including shoulder and arm position) of the patient residing on the mattress. Furthermore, by providing said piezoelectric sensor strip covering above-mentioned width of the mattress, the PMS is able to collect information regarding physiological parameters of the patient residing on the mattress, such as breathing amplitude and heart rate of the patient.

The sensing assembly of the PMS of the current invention further comprises a sensor module which receives the electrical signals from the plurality of sensors and converts them to digital signals. In an embodiment, all sensor entities (the piezoelectric sensor strip, the sensor grid and the linearly extending pressure sensors) are alle provided with the same type of connector. In an embodiment, said sensors comprise a female connector able to connect with a male connector of the sensor module. The signals from the plurality of sensors are read out by one or more methods. In an embodiment, the discrete pressure sensors of the sensor grid are read out by means of a zero potential scanning method. In an embodiment, the linearly extending pressure sensors are read out according to a regular resistance measurement. In an exemplary embodiment, the sensor module may be disposed in the interior of the mattress. The sensor module transfers the signals to a control unit.

The control unit includes at least a memory and a processing unit. The control unit may be electrically coupled to the sensor module. In an embodiment, the control unit is placed under the frame of the bed where the mattress is placed.

The control unit may be embodied as one or more microprocessors with accompanying digital signal processor(s), one or more processor(s) without an accompanying digital signal processor, one or more coprocessors, one or more multicore processors, one or more controllers, processing circuitry, one or more computers, various other processing elements including integrated circuits such as, for example, an application specific integrated circuit (ASIC) or field programmable gate array (FPGA), or some combination thereof.

Accordingly, in an example embodiment, the processing unit may include a plurality of processors and signal processing modules. The plurality of processors may be in operative communication with each other and may be collectively configured to perform one or more functionalities of the circuitry of the control system, as described herein. In an example embodiment, the control unit may be configured to execute instructions stored in the memory device or otherwise accessible to the control unit. These instructions, when executed by the control unit, may cause the circuitry of the mattress to perform one or more of the functionalities, as described herein.

Whether configured by hardware, firmware/software methods, or by a combination thereof, the control unit may include an entity capable of performing operations according to embodiments of the present disclosure while configured accordingly. Thus, for example, when the control unit is embodied as an ASIC, FPGA or the like, the control unit may include specifically configured hardware for conducting one or more operations described herein. Alternatively, as another example, when the control unit is embodied as an executor of instructions, such as may be stored in the memory device, the instructions may specifically configure the control unit to perform one or more algorithms and operations described herein.

Thus, the control unit used herein may refer to a programmable microprocessor, microcomputer or multiple processor chip or chips that can be configured by software instructions (applications) to perform a variety of functions, including the functions of the various embodiments described above.

Multiple processors may be provided dedicated to wireless communication functions and one processor dedicated to running other applications. Software applications may be stored in the internal memory before they are accessed and loaded into the processors. The processors may include internal memory sufficient to store the application software instructions. In many devices, the internal memory may be a volatile or nonvolatile memory, such as flash memory, or a mixture of both. The memory can also be located internal to another computing resource (e.g., enabling computer readable instructions to be downloaded over the Internet or another wired or wireless connection).

Further, the PMS includes a communication unit that communicatively connects the control unit to at least one monitoring station. In an embodiment, the control unit is configured to generate an alarm signal based on data received from the plurality of sensors.

As such, the PMS may include an alarm which may be triggered based on information received from either the control unit or a monitoring station. Further, in an embodiment, the PMS includes at least one display, which can be positioned in proximity to the bed or at amonitoring station, to convey information received from the control unit or from amonitoring station.

In an embodiment, the PMS comprises a RFID scanner. In a further embodiment the RFID scanner is equipped to scan digital codes, such as those present on personnel badges. In an embodiment, the PMS comprises a handset to process said data from said RFID scanner. In an embodiment the data from said RFID scanner can be linked to certain operations, for instance operations linked to the PMS, but also operations linked to external devices. In an embodiment, the digital codes present on personnel badges are linked to instructions for elevating of lowering the height of the bed on which the mattress of the PMS resides. In an embodiment, the handset comprises a display to show data (such as warning messages, error displays, etc.) and one or more buttons for operating one or more (external) devices.

The communication unit includes a transceiver, where the transceiver may correspond to a communication interface that may facilitate transmission and reception of messages and data to and from various devices. Examples of the transceiver may include, but are not limited to, an antenna, an Ethernet port, a USB port, a serial port, or any other port that can be adapted to receive and transmit data. The transceiver transmits and receives data and/or messages in accordance with the various communication protocols, such as, Digital Enhanced Cordless Telecommunications (DECT) for local transmission of alarms, Bluetooth^{®}, Infra-Red, I2C, TCP/IP, UDP, and 2G, 3G, 4G or 5G communication protocols.

In a preferred embodiment, the control unit is communicatively coupled to a local monitoring station (such as a local alarm server) by means of Wifi/LAN/DECT/Relays/Bluetooth^{®}. In an embodiment, the control unit comprises Wifi/LAN/DECT/ Bluetooth^{®} capability to connect to said local monitoring station. In an embodiment, said local monitoring station is a nurse call system. In an embodiment, the control unit comprises multiple relays for local switching of alarms.

In a preferred embodiment, the control unit is communicatively coupled to a remote monitoring station (such as a server) by means of a low-power wide-area-network, such as an LTE-M module. Said LTE-M module allows remote assistance and guaranteed connection for alarms. In an embodiment, said remote monitoring station allows to remotely connect to a helpdesk or to send messages such as SMS. In an embodiment, the control unit is communicatively coupled to a remote monitoring station (for instance by a LPWAN network), which is communicatively coupled to a local monitoring station.

In an embodiment, said Bluetooth^{®} capability allows for connecting with additional sensors. In an embodiment, the control unit further comprises expansion connectors for addition of new sensors. In an embodiment, the control unit comprised a built-in battery that allows for detection and override of a short power loss.

As described above, the PMS according to the invention comprises a mattress wherein the plurality of sensors is disposed in the mattress itself. In an embodiment, said mattress of the PMS comprises a first layer and one or more additional layers, the first layer being the top layer of the mattress being closest to the patient when the latter is present, the plurality of sensors being disposed between said layers of the mattress. In an embodiment, one or more of the layers are adapted to receive the plurality of sensors (and associated wiring) and/or the sensor module, for instance by providing recesses in the material of which the layers are made, wherein said recesses are able to incorporate the plurality of sensors (and associated wiring) and/or the sensor module in the interior of the layer material. In an embodiment, the mattress comprises of a first utmost layer (top layer) and a second utmost layer (on the opposite side of the top layer) and two layers to which the plurality of sensors are attached, said two layers being positioned between said first and second utmost layers. By disposing the plurality of sensors between said layers of the mattress, movement of the sensors is limited and the specific location of the plurality of sensors with regard to the mattress is confined. As described above, the specific location of the plurality of sensors with regard to the mattress is important to accurately determine the posture of a patient residing on the mattress of the PMS. Furthermore, disposing the plurality of sensors between said layers, allows for more easy handling of the sensors, minimizing the risk to damage or lose one or more of the provided sensors, extending the product life of the mattress (and the PMS). The mattress of the current invention can be made from every material known from the state of the art, such as foam (including polyurethane foam, latex foam, memory foam), polystyreen, natural fibers or any combination of materials.

In an embodiment, the thickness of the first layer and the joint thickness of the one or more additional layers relate to each other with a ratio comprising between 45% and 80%. By providing a PMS comprising a mattress comprising such a ratio, an optimal balance between the sensitivity of the sensors (not a too thick first layer) and the comfort of the patient (not a too thin first layer) and the overall optimal thickness of the mattress (not too thin, to allow an optimal degree of comfort for the patient and not too thick, to allow an optimal degree of flexibility of the mattress) can be achieved.

In an embodiment, the plurality of sensors further comprises one or more temperature sensors. In a preferred embodiment, the sensor module comprises a temperature sensor for monitoring the temperature of the hardware of the sensor module.

In an embodiment, a temperature sensor may also be disposed in the interior of the mattress to sense the temperature of the foam/cushion of the mattress. The signals generated by the temperature sensor help in determining the condition of the mattress. Additionally, the temperature sensor may be configured to determine the temperature of the patient lying on the mattress.

In a second aspect, the current invention relates to a mattress for patient monitoring, the mattress comprising:
a first layer and one or more additional layers; and
a sensing assembly disposed between said layers of the mattress, said sensing assembly comprising:
   a plurality of sensors, wherein the plurality of sensors transmit signals and comprise:
      one or more piezoelectric sensor strips;
      a sensor grid comprising a plurality of discrete pressure sensors arranged in one or more rows, each of the one or more rows being coupled with each other through a conducting element, wherein the one or more piezoelectric sensor strips and the sensor grid are disposed in proximity to a first edge of the mattress;
      one or more linearly extending pressure sensors extending along at least a part length of or a full length of the mattress, wherein the one or more linearly extending pressure sensors are placed in parallel with and in proximity of a third edge and/or a fourth edge of the mattress, wherein the third edge and the fourth edge of the mattress are spaced apart from each other along a lateral axis of the mattress, said linearly extending pressure sensors covering at least 50% of the length of the lower half of the mattress measured according to the longitudinal axis of the mattress; and
   a sensor module for receiving said signals from said sensors.

In a third aspect the present disclosure relates to a method for monitoring a patient using a patient monitoring system or a mattress according to one of the previous embodiments, the method comprises:
a. receiving piezoelectric sensor data from one or more piezoelectric sensor strips indicating body motion of the patient;
b. receiving force sensor data from the sensor grid indicating a position of the patient on the mattress;
c. differentiation between body motion that involves a posture change of the patient and body motion that does not involve a posture change of the patient based on the piezoelectric sensor data and the force sensor data; and
d. generating an alarm signal when no posture change has occurred during a time greater than a certain threshold.

At step a of the method, piezoelectric sensor data is received from the one or more piezoelectric sensor strips. Said data is used to determine whether the patient is present or not on the mattress and, when the patient is present on the mattress, whether movement of the patient has taken place.

In one embodiment, the control unit may be configured to receive the piezoelectric sensor data periodically. In another embodiment, the control unit may receive the piezoelectric sensor data when a change in movement and/or in one or more of the physiological parameters associated with the patient is detected. In such an embodiment, the one or more piezoelectric sensor strips may be configured to determine the change in movement and/or in one or more the physiological parameters of the patient. In an event of the change, the one or more piezoelectric sensor strips may be configured to transmit the piezoelectric sensor data. To detect the change in the movement or in one or more physiological parameters, the one or more piezoelectric sensor strips may be configured to utilize known technologies such as, but not limited to, moving average, mean, mode, median, and/or the like.

At step b of the method, the force sensor data is received from the sensor grid. In an exemplary embodiment, the control unit may be configured to receive the force sensor data from the sensor grid. In an exemplary embodiment, the force sensor data may be indicative of the position of the patient on the mattress. Similar to the one or more piezoelectric sensor strips, the sensor grid may be configured to transmit the force sensor data.

At step c, differentiation between body motion that involves a posture change of the patient and body motion that does not involve a posture change of the patient based on the piezoelectric sensor data and the force sensor data is performed.

At step d of the method an alarm signal is generated when no posture change has occurred during a time greater than a certain threshold.

In a further embodiment of the abovementioned method, the control unit may be configured to derive the respiratory rate and the heart rate based on the piezoelectric sensor data. Further, the control unit may be configured to determine force value from the force sensor data. Thereafter, in an exemplary embodiment, the control unit may be configured to determine the posture of the patient based on a look up table 1 below.

**Table 1: determination of the posture of the patient based on the force sensor data and the piezoelectric sensor data**

| Force sensor data | Respiration rate (breath/min) | Heartbeat (beats/min) | Posture |
|---|---|---|---|
| 24 (newton) | 20 | 60 | Supine |
| 50 (newton) | 30 | 80 | Prone |
| 100 (newton) | 40 | 100 | Foetal |

In some examples, the control unit may be configured to detect the potential development of decubitus ulcers (bed sores). To this end, the control unit may be configured to determine time-lapsed since the patient is in a certain posture. Thereafter, the control unit may be configured to compare the time lapsed with a certain time threshold. If the control unit determines that the time lapsed is greater than the time threshold, the control unit may be configured to prevent a potential onset of bed sores. To this end, the control unit may be configured to generate an alarm signal. In some examples, the monitoring station may be configured to inform the requisite hospital staff upon reception of the alarm signal to take necessary action. Further, the control unit may be configured to transmit the alarm signal to the monitoring station. However, if the control unit determine the time lapsed is less than the time threshold, the control unit may be configured to repeat the step.

In an embodiment a potential patient fall condition is detected based on the pressure sensor data received from the linearly extending pressure sensors positioned proximate to lateral edges of the mattress. In an exemplary embodiment, the control unit may be configured to detect the potential patient fall condition when the control unit receives the data from the linearly extending pressure sensors positioned proximate to lateral edges of the mattress. Reception of the data from the pressure sensor may indicate that patient is on the edge of the mattress and may fall. Upon reception of the pressure sensor data, the control unit may be configured to generate an alarm signal.

In an embodiment, the monitoring station may be configured to receive temperature information from the one or more temperature sensors in order to determine the temperature of the hardware comprised in the sensor module, the condition of the mattress and/or the temperature of the patient residing on the mattress.

In an embodiment, the method further comprises determining physiological parameters of the patient based on the piezoelectric sensor data from one or more of the piezoelectric sensor strips. As described above, the piezo-electric sensor allows to measure heartbeat-induced repeated motions of the human body (measured by a technique called ballistocardiography) and changes in load or center of gravity due to breathing of the patient.

In an embodiment of the method, said physiological parameters are used to verify the position of a patient.

In an embodiment, said signals when assessed over an extended time period may be indicative of the position of a patient, whereas a high breathing amplitude and heart rate of a patient are indicative for abdominal position of the patient, a median breathing amplitude and high heart rate are indicative for left side position of the patient and a median breathing amplitude and low heart rate are indicative for a rightside position of the patient.

In an embodiment, said signals may further be used to monitor sleeping pattern of the patient that further includes duration, a phase, or a quality of sleep of the patient.

In an embodiment, said signals from the one or more piezoelectric sensor strips are used to detect breathing disorders, such as sleep-disordered breathing (SDB), better known as obstructive sleep apnea (OSA) syndrome. OSA and associated cardiovascular complications are among the most common clinical disorders. The ballistocardiogram (BCG) signal measured by the one or more piezoelectric sensor strips captures the ballistic forces of the heart caused by the sudden ejection of blood into the great vessels with each heartbeat, breathing, and body movement.

In an embodiment, cardiac function and performance is measured by the one or more piezoelectric strips.

In an exemplary embodiment, the control unit may be configured to detect breathing disorders. For example, the control unit may be configured to monitor the respiration rate while the patient is sleeping. If the control unit determines that the respiration rate is below a predetermined threshold, the control unit may be configured to generate an alarm signal indicative of onset of breathing disorder. In some examples, the control unit may be configured to transmit the alarm signal to a monitoring station. In some examples, the scope of the disclosure is not limited to the control unit generating the alarm signal when the respiration rate is less than the predetermined threshold. In an alternative embodiment, the control unit may be configured to generate the alarm signal when the respiration rate is greater than the predetermined threshold. In some examples, a monitoring station may be configured to inform the requisite hospital staff upon reception of the alarm signal to take necessary action.

### DESCRIPTION OF FIGURES

The present invention is in no way limited to the given embodiments presented in the figures.

Figure 1 depicts a schematic lay-out of the mattress **1** of the PMS (not shown) according to an embodiment of the current invention. The mattress comprises a sensor assembly comprising a plurality of sensors which transmit signals and a sensor module **2** for receiving said signals. The plurality of sensors and the sensor module **2** are mounted within the mattress **1** (however, for clarity reasons, the elements of the sensor assembly are depicted on top of the mattress in figure 1).

In this specific embodiment, the mattress comprises one piezoelectric sensor strip **3**; a sensor grid **4** comprising a plurality of discrete pressure sensors and a pair of linearly extending pressure sensors **5a,5b.** The piezoelectric sensor strip **3** and the sensor grid **4** are disposed in proximity to a first edge **6** of the mattress **1.** The linearly extending pressure sensors **5a,5b** are placed in parallel with and in proximity to a third edge **7** and a fourth edge **8** of the mattress **1,** wherein the third edge and the fourth edge of the mattress are spaced apart from each other along the lateral axis **9** of the mattress **1** and the pair of linearly extending pressure sensors **5a,5b** are positioned proximate to lateral edges **7,8** of the mattress **1.** The linearly extending pressure sensors **5a,5b** (more specifically force sensing resistors) cover each approximately at least 60% of the length of the lower half of the mattress measured according to the longitudinal axis **10** of the mattress **1.** As such, the linearly extending pressure sensors **5a,5b** allow to collect spatially relevant information about pressure points covering at least a part of the lateral edges **7,8** of the lower halve of the mattress **1,** indicating whether a patient (not shown) might be exiting the bed (not shown).

The mattress **1** of the PMS comprises one piezoelectric sensor strip **3** extending laterally across a part of the width of the mattress **1.** Said piezoelectric sensor strip **1** is located between the second **12** and third row **13** of the sensor grid **4** and covers approximately 77% of the width of the mattress **1** measured according to the lateral axis **9** of the mattress 1. By providing said piezoelectric sensor strip **3** covering above-mentioned width of the mattress **1,** said piezoelectric sensor strip **3** allows to detect body movement of the patient residing on the mattress **1.** Furthermore, by providing said piezoelectric sensor strip **3** covering above-mentioned width of the mattress **1,** the PMS is able to collect information regarding physiological parameters of the patient residing on the mattress, such as breathing amplitude and heart rate of the patient. Said signals when assessed over an extended time period may be indicative of the position of a patient, whereas a high breathing amplitude and heart rate of a patient are indicative for abdominal position of the patient, a median breathing amplitude and high heart rate are indicative for left side position of the patient and a median breathing amplitude and low heart rate are indicative for a rightside position of the patient. Said signals may further be used to detect breathing disorders, such as sleep-disordered breathing (SDB).

The sensor grid **4** comprises 16 discrete pressure sensors **16,** more specifically force sensing resistors, arranged in a first **11,** second **12** and third row **13.** The first row **11** is closest to the first edge **6** of the mattress, the second row **12** is placed in between the first **11** and third row **13** and the third row **13** is closest to the second edge **15** of the mattress **1.** The three rows **11,12, 13** comprise respectively 7, 6 and 3 discrete pressure sensors **16.** The pressure sensors **16** of the first row **11** are arranged in such a manner that the pressure sensor closest to the third edge **7** and the pressure sensor closest to the fourth edge **8** of the mattress are spaced a minimal distance apart, said minimal distance covering approximately 66% of the width of the mattress measured according to the lateral axis of the mattress **9.** The pressure sensors **16** of the second row **12** are arranged in such a manner that the pressure sensor **16** closest to the third edge **7** and the pressure sensor closest to the fourth edge **8** are spaced a minimal distance apart, said minimal distance covering approximately 55% of the width of the mattress **1** measured according to the lateral axis **9** of the mattress **1.** The pressure sensors **16** of the third row **13** are arranged in such a manner that the pressure sensor **16** closest to the third edge **7** and the pressure sensor closest to the fourth edge **8** are spaced a minimal distance apart, said minimal distance covering approximately 44 % of the width of the mattress **1** measured according to the lateral axis **9** of the mattress **1.** By providing said specific configuration of pressure sensors **16** in the sensor grid **4,** covering above-mentioned width of the mattress **1,** the PMS is able to collect spatially relevant information about pressure points covering the torso region, which allows for an accurate position classification of the patient residing on the mattress. The sensor grid **4** allows to collect information about pressure points in the torso region after which an algorithm gives a position classification. This classification will not be directly linked to a posture but is purely for determining whether there is a new posture measured after the piezo sensor indicates that a movement has taken place.

The sensing assembly of the PMS further comprises a sensor module **2** which receives the electrical signals by means of analog wires **17** from the plurality of sensors and converts them to digital signals. The sensor module **2** further comprises a temperature sensor (not shown) for monitoring the temperature of the hardware comprised in the sensor module.

## Claims

1. A mattress **1** for patient monitoring, the mattress **1** having a first edge **6** and a second edge **15,** wherein the first edge **6** and the second edge **15** are spaced apart from each other along a longitudinal axis **10** of the mattress **1,** the mattress **1** comprising a sensor assembly, said sensor assembly comprising:
- a plurality of sensors disposed in the mattress, wherein the plurality of sensors transmit signals and comprise:
one or more piezoelectric sensor strips **3**;
a sensor grid **4** comprising a plurality of discrete pressure sensors **16** arranged in one or more rows **11,12,13,** each of the one or more rows **11,12,13** being coupled with each other through a conducting element, wherein the one or more piezoelectric sensor strips **3** and the sensor grid **4** are disposed in proximity to the first edge **6** of the mattress **1**;
one or more linearly extending pressure sensors **5a,5b** extending along at least a part length of or a full length of the mattress **1,** wherein the one or more linearly extending pressure sensors **5a,5b** are placed in parallel with and in proximity of a third edge **7** and/or a fourth edge **8** of the mattress **1,** wherein the third edge **7** and the fourth edge **8** of the mattress **1** are spaced apart from each other along a lateral axis **9** of the mattress **1,** said linearly extending pressure sensors **5a,5b** covering at least 50% of the length of the lower half of the mattress **1,** measured according to the longitudinal axis **10** of the mattress **1**;
- a sensor module **2** for receiving said signals from said sensors **3,4,5,** wherein the sensor module **2** is mounted within the mattress **1.**

2. The mattress according to claim 1, wherein the sensor grid **4** comprises at least 12 discrete pressure sensors **16** arranged in one or more rows, preferably 16 discrete pressure sensors **16,** arranged in a first **11,** second **12** and third row **13,** said rows **11,12,13** extending laterally across a part of or across a total width of the mattress **1,** the first row **11** being closest to the first edge **6** of the mattress **1,** the second row **12** being placed in between the first **11** and third row **13** and the third row **13** being closest to the second edge **15** of the mattress, the three rows **11,12,13** comprising respectively 7, 6 and 3 discrete pressure sensors **16** and wherein the pressure sensors **16** of the first row **11** are arranged in such a manner that the pressure sensor **16** closest to the third edge **7** and the pressure sensor closest to the fourth edge **8** are spaced a minimal distance apart, said minimal distance covering at least 60% of the width of the mattress **1** measured according to the lateral axis **9** of the mattress **1.**

3. The mattress according to claim 2, the mattress **1** comprising one piezoelectric sensor strip **3** extending laterally across a part of or across a total width of the mattress **1,** said piezoelectric sensor strip **3** being located between the second **12** and third row **13** of the sensor grid **4** and covering at least 65% of the width of the mattress **1** measured according to the lateral axis **9** of the mattress 1.

4. The mattress according to any of the previous claims, wherein the mattress 1 comprises a first layer and one or more additional layers, the first layer being the top layer of the mattress being closest to the patient when the latter is present, wherein the plurality of sensors **3,4,5** are disposed between said layers of the mattress.

5. The mattress according to claim 4, wherein the thickness of the first layer and the joint thickness of the one or more additional layers relate to each other with a ratio comprising between 45% and 80%.

6. The mattress according to any of the previous claims, wherein the plurality of sensors **3,4,5** further comprises one or more temperature sensors.

7. A patient monitoring system, the system comprising:
- a mattress **1** according to any of the previous claims 1-6,
- a control unit electrically coupled to the sensor module **2** for processing said signals, and at least one monitoring station communicatively coupled to the control unit.

8. The patient monitoring system according to claim 7, wherein the control unit is configured to generate an alarm signal based on data received from the plurality of sensors **3,4,5.**

9. The patient monitoring system according to any of the previous claims 7-8, wherein the control unit is communicatively coupled to a remote monitoring station by means of a low-power wide-area-network, such as an LTE-M module.

## Patentansprüche

1. Matratze **1** zur Patientenüberwachung, wobei die Matratze **1** einen ersten Rand **6** und einen zweiten Rand **15** aufweist, wobei der erste Rand **6** und der zweite Rand **15** entlang einer Längsachse **10** der Matratze **1** voneinander beabstandet sind, wobei die Matratze **1** eine Sensoranordnung umfasst und die Sensoranordnung Folgendes umfasst:
- mehrere Sensoren, die in der Matratze angeordnet sind, wobei die mehreren Sensoren Signale übertragen und Folgendes umfassen:
einen oder mehrere piezoelektrische Sensorstreifen **3,**
ein Sensorgitter **4,** das mehrere einzelne Drucksensoren **16** umfasst, die in einer oder mehreren Reihen **11, 12, 13** angeordnet sind, wobei jede der einen oder mehreren Reihen **11, 12, 13** durch ein leitendes Element miteinander gekoppelt sind, wobei der eine oder die mehreren piezoelektrischen Sensorstreifen **3** und das Sensorgitter **4** nahe dem ersten Rand **6** der Matratze **1** angeordnet sind,
einen oder mehrere sich linear erstreckende Drucksensoren **5a, 5b,** die sich entlang mindestens eines Teils der Länge oder einer gesamten Länge der Matratze **1** erstrecken, wobei der eine oder die mehreren sich linear erstreckenden Drucksensoren **5a, 5b** parallel zum und nahe einem dritten Rand **7** und/oder einem vierten Rand **8** der Matratze 1 platziert sind, wobei der dritte Rand **7** und der vierte Rand **8** der Matratze **1** entlang einer Querachse **9** der Matratze **1** voneinander beabstandet sind, wobei die sich linear erstreckenden Drucksensoren **5a, 5b** mindestens 50 % der Länge der unteren Hälfte der Matratze **1** abdecken, gemessen gemäß der Längsachse **10** der Matratze **1,**
- ein Sensormodul **2** zum Empfangen der Signale von den Sensoren **3**, **4, 5,** wobei das Sensormodul **2** in der Matratze **1** montiert ist.

2. Matratze nach Anspruch 1, wobei das Sensorgitter **4** mindestens 12 einzelne Drucksensoren **16** umfasst, die in einer oder mehreren Reihen angeordnet sind, vorzugsweise 16 einzelne Drucksensoren **16,** die in einer ersten **11,** einer zweiten **12** und einer dritten Reihe **13** angeordnet sind, wobei sich die Reihen **11, 12, 13** quer über einen Teil der Breite oder eine gesamte Breite der Matratze **1** erstrecken, wobei die erste Reihe **11** dem ersten Rand **6** der Matratze **1** am nächsten liegt, die zweite Reihe **12** zwischen der ersten **11** und der dritten Reihe **13** platziert ist und die dritte Reihe **13** dem zweiten Rand **15** der Matratze **1** am nächsten liegt, wobei die drei Reihen **11, 12, 13** jeweils 7, 6 und 3 einzelne Drucksensoren **16** umfassen und wobei die Drucksensoren **16** der ersten Reihe **11** derart angeordnet sind dass der Drucksensor **16,** der dem dritten Rand **7** am nächsten liegt, und der Drucksensor, der dem vierten Rand **8** am nächsten liegt, in einer Mindestdistanz beabstandet sind, wobei die Mindestdistanz mindestens 60 % der Breite der Matratze **1** abdeckt, gemessen gemäß der Querachse **9** der Matratze **1** .

3. Matratze nach Anspruch 2, wobei die Matratze **1** einen piezoelektrischen Sensorstreifen **3** umfasst, der sich quer über einen Teil einer Breite oder über eine gesamte Breite der Matratze **1** erstreckt, wobei sich der piezoelektrische Sensorstreifen **3** zwischen der zweiten **12** und der dritten Reihe **13** des Sensorgitters **4** befindet und mindestens 65 % der Breite der Matratze 1 abdeckt, gemessen gemäß der Querachse **9** der Matratze **1.**

4. Matratze nach einem der vorhergehenden Ansprüche, wobei die Matratze **1** eine erste Schicht und eine oder mehrere zusätzliche Schichten umfasst, wobei die erste Schicht, welche die obere Schicht der Matratze ist, dem Patienten am nächsten ist, wenn letzterer anwesend ist, wobei die mehreren Sensoren **3, 4, 5** zwischen den Schichten der Matratze angeordnet sind.

5. Matratze nach Anspruch 4, wobei die Dicke der ersten Schicht und die Gesamtdicke der einen oder mehreren zusätzlichen Schichten zueinander in einem Verhältnis zwischen 45 % und 80 % stehen.

6. Matratze nach einem der vorhergehenden Ansprüche, wobei die mehreren Sensoren **3, 4, 5** ferner einen oder mehrere Temperatursensoren umfassen.

7. Patientenüberwachungssystem, wobei das System Folgendes umfasst:
- eine Matratze **1** nach einem der vorhergehenden Ansprüche 1 bis 6,
- eine Steuereinheit, die elektrisch mit dem Sensormodul **2** verbunden ist, um die Signale zu verarbeiten, und mindestens eine Überwachungsstation, die kommunikativ mit der Steuereinheit gekoppelt ist.

8. Patientenüberwachungssystem nach Anspruch 7, wobei die Steuereinheit dafür konfiguriert ist, basierend auf Daten, die von den mehreren Sensoren **3, 4, 5** empfangen werden, ein Alarmsignal zu erzeugen.

9. Das Patientenüberwachungssystem nach einem der vorhergehenden Ansprüche 7 bis 8, wobei die Steuereinheit über ein Niedrigenergieweitverkehrsnetz, wie beispielsweise ein LTE-M-Modul, kommunikativ mit einer entfernten Überwachungsstation gekoppelt ist.

## Revendications

1. Matelas **1** permettant la surveillance d'un patient, le matelas **1** possédant un premier bord **6** et un deuxième bord **15,** dans lequel le premier bord **6** et le deuxième bord **15** sont espacés l'un de l'autre le long d'un axe longitudinal **10** du matelas **1,** le matelas **1** comprenant un ensemble de détection, ledit ensemble de détection comprenant:
- une pluralité de capteurs disposés dans le matelas, dans lequel la pluralité de capteurs transmet des signaux et comprend:
une ou plusieurs bandes de détection piézoélectriques **3**;
une grille de détection **4** comprenant une pluralité de capteurs de pression discrets **16** disposés dans une ou plusieurs rangées **11,12,13,** chacune des une ou plusieurs rangées **11,12,13** étant couplée les unes aux autres par l'intermédiaire d'un élément conducteur, dans lequel la ou les bandes de détection piézoélectriques **3** et la grille de détection **4** sont disposées à proximité du premier bord **6** du matelas **1;**
un ou plusieurs capteurs de pression s'étendant linéairement **5a, 5b** s'étendant le long d'au moins une longueur partielle du matelas **1** ou sur toute sa longueur, dans lequel le ou les capteurs de pression s'étendant linéairement **5a, 5b** sont placés parallèlement et à proximité d'un troisième bord **7** et/ou d'un quatrième bord **8** du matelas **1,** dans lequel le troisième bord **7** et le quatrième bord **8** du matelas **1** sont espacés l'un de l'autre le long d'un axe latéral **9** du matelas **1,** lesdits capteurs de pression s'étendant linéairement **5a, 5b** couvrant au moins 50 % de la longueur de la partie inférieure du matelas **1,** mesurée en fonction de l'axe longitudinal **10** du matelas **1;**
- un module de détection **2** pour recevoir lesdits signaux à partir desdits capteurs **3, 4, 5,** dans lequel le module de détection **2** est monté à l'intérieur du matelas **1.**

2. Matelas selon la revendication **1,** dans lequel la grille de détection **4** comprend au moins 12 capteurs de pression discrets **16** disposés dans une ou plusieurs rangées, de préférence 16 capteurs de pression discrets **16,** disposés dans une première **11,** une deuxième **12,** et une troisième rangée **13,** lesdites rangées **11,12,13** s'étendant latéralement sur une partie de la largeur du matelas **1** ou sur toute sa largeur, la première rangée **11** étant la plus proche du premier bord **6** du matelas **1,** la deuxième rangée **12** étant placée entre la première **11** et la troisième rangée **13,** et la troisième rangée **13** étant la plus proche du deuxième bord **15** du matelas, les trois rangées **11,12,13** comprenant respectivement 7, 6 et 3 capteurs de pression discrets **16** et dans lequel les capteurs de pression **16** de la première rangée **11** sont disposés de telle sorte que le capteur de pression **16** le plus proche du troisième bord **7** et le capteur de pression le plus proche du quatrième bord **8** sont espacés d'une distance minimale, ladite distance minimale couvrant au moins 60% de la largeur du matelas **1** mesurée selon l'axe latéral **9** du matelas **1.**

3. Matelas selon la revendication 2, le matelas **1** comprenant une bande de détection piézoélectrique **3** s'étendant latéralement sur une partie de la largeur du matelas **1** ou sur toute sa largeur, ladite bande de détection piézoélectrique **3** étant située entre la deuxième **12** et la troisième rangée **13** de la grille de détection **4** et couvrant au moins 65% de la largeur du matelas **1** mesurée selon l'axe latéral **9** du matelas **1.**

4. Matelas selon l'une quelconque des revendications précédentes, dans lequel le matelas **1** comprend une première couche et une ou plusieurs couches supplémentaires, la première couche étant la couche supérieure du matelas la plus proche du patient lorsque ce dernier est présent, dans lequel la pluralité de capteurs **3,4,5** sont disposés entre lesdites couches du matelas.

5. Matelas selon la revendication 4, dans lequel l'épaisseur de la première couche et l'épaisseur commune de la ou des couches supplémentaires sont liées entre elles dans un rapport compris entre 45% et 80%.

6. Matelas selon l'une quelconque des revendications précédentes, dans lequel la pluralité de capteurs **3,4,5** comprend en outre un ou plusieurs capteurs de température.

7. Système de surveillance de patients, le système comprenant:
- un matelas **1** selon l'une quelconque des revendications précédentes 1 à 6,
- une unité de commande couplée électriquement au module de détection **2** pour traiter lesdits signaux, et au moins une station de surveillance couplée en communication à l'unité de commande.

8. Système de surveillance de patients selon la revendication 7, dans lequel l'unité de commande est configurée pour générer un signal d'alarme sur la base de données reçues à partir de la pluralité de capteurs **3,4,5.**

9. Système de surveillance de patients selon l'une quelconque des revendications 7 et 8 précédentes, dans lequel l'unité de commande est couplée en communication à une station de surveillance à distance au moyen d'un réseau étendu de faible puissance, tel qu'un module LTE-M.
